# EUROPEAN PATENT APPLICATION

(11) **EP 4 718 463 A1**
(43) Date of publication of application: **01.04.2026**
(21) Application number: 24203440.3
(22) Date of filing: 30.09.2024
(51) Int. Cl.: G16H 20/30, A46B 15/00

(54) **ORAL CARE ASSESSMENT**

(71) Applicant: Koninklijke Philips N.V., 5656 AG Eindhoven (NL)
(72) Inventor: SCHEFFERS, Lucas Petrus Henricus, Eindhoven (NL); GOTTENBOS, Bart, Eindhoven (NL)
(74) Representative: Philips Intellectual Property & Standards

(57) **Abstract**

Proposed concepts thus aim to provide schemes, solutions, concepts, designs, methods, and systems pertaining to assessing performance of an oral health care routine. In particular, embodiments propose to utilise data from one or more sensors to detect specific dental features (e.g. particularly interproximal (IP) spaces between teeth). Such sensors may include pressure sensors, inertial measurement units (IMUs), or optical sensors, each capable of identifying patterns created by dental features. By analysing the data from such sensors, embodiments may, for example, effectively count the number of teeth scanned and assess the completeness of the performance of an oral health care routine.

## Description

### FIELD OF THE INVENTION

The present invention relates to oral care assessment systems and methods, and more particularly to systems and methods for analysing sensor data from oral care devices to evaluate the quality and completeness of oral care routines.

### BACKGROUND OF THE INVENTION

Intraoral scanning has become an increasingly important tool in modern dentistry, offering numerous applications ranging from diagnostic imaging to treatment planning. These scanners capture detailed digital impressions of a patient's oral cavity, providing valuable data for various dental procedures and assessments. As the technology has advanced, intraoral scanners have been integrated with additional sensors and imaging capabilities, expanding their potential uses in oral health monitoring and preventive care.

Despite these advancements, ensuring complete and accurate coverage during intraoral scanning remains a significant challenge. Users, whether dental professionals or patients using at-home devices, may inadvertently miss certain areas or fail to capture all necessary dental surfaces. This incomplete coverage can lead to inaccurate diagnoses, suboptimal treatment plans, or missed early signs of oral health issues. Additionally, the large amount of data generated by high-resolution intraoral scans can strain processing capabilities and storage systems, particularly in portable or consumer-grade devices.

Quality control measures for intraoral scanning are often limited, relying primarily on visual inspection or basic position tracking. These methods may not provide sufficient accuracy or real-time feedback to ensure comprehensive coverage of all dental surfaces. Furthermore, existing systems typically lack the ability to automatically assess the completeness and quality of a scan, potentially leading to repeated procedures or overlooked areas of concern.

### SUMMARY OF THE INVENTION

The invention is defined by the claims.

In a first aspect, a method for assessing an oral health care routine performed using an oral care device is provided. The method comprises obtaining routine performance data describing at least one of: motion of the oral care device and pressure applied to the oral care device, during performance of the oral health care routine; analysing the routine performance data to identify one or more instances of a dental feature covered during performance of the oral health care routine; and analysing the identified one or more instances of a dental feature to determine a parameter value of the oral health care routine.

Proposed concepts thus aim to provide schemes, solutions, concepts, designs, methods, and systems pertaining to assessing performance of an oral health care routine. In particular, embodiments propose to utilise data from one or more sensors to detect specific dental features (e.g. particularly interproximal (IP) spaces between teeth). Such sensors may include pressure sensors, inertial measurement units (IMUs), or optical sensors, each capable of identifying patterns created by dental features. By analysing the data from such sensors, embodiments may, for example, effectively count the number of teeth scanned and assess the completeness of the performance of an oral health care routine.

Example of the proposed invention may provide a teeth counting method/system for oral/dental cameras or intraoral scanners, enhancing quality control in oral health imaging and diagnostics (such as measuring or imaging routines). Proposed embodiments may leverage sensors and data processing techniques to accurately count teeth during scanning procedures (e.g. oral measuring or imaging routines), ensuring comprehensive coverage and improving the overall quality of oral scans.

Proposed embodiments may utilise data from one or more sensors to detect specific dental features, particularly interproximal (IP) spaces between teeth. These sensors may include pressure sensors, inertial measurement units (IMUs), or optical sensors, each capable of identifying the repetitive patterns created by teeth and the spaces between them. By analysing the data from these sensors, embodiments may, for example, effectively count the number of teeth scanned and assess the completeness of the oral health care routine.

Embodiments may employ data processing to analyse sensor information to generate a parameter value (e.g. completion measure or quality score) for an oral health care routine. This parameter value may be based on factors such as the number of teeth detected, the consistency of the scanning motion, and the coverage of different areas within the mouth. By comparing the calculated parameter value against predetermined thresholds, embodiments may automatically accept or reject performed routines (e.g. oral scans), ensuring that only high-quality, complete care routines (e.g. oral scans) are used for further analysis or diagnosis.

A key advantage of the proposed concept is its ability to provide real-time feedback to users. By detecting missed areas or incomplete oral health care routines, embodiments can guide users to repeat the performance of all or part of an oral health care routine (e.g. instruct to re-scan specific regions), improving the overall quality and completeness of the oral health care routine. This may be particularly valuable in applications such as plaque detection, stain assessment, and caries identification, where comprehensive coverage is crucial for accurate diagnosis and treatment planning.

Furthermore, the proposed teeth counting capability of some embodiments may offer potential benefits beyond quality control. For example, by accurately tracking the number and position of teeth, embodiments could assist in monitoring dental health over time, identifying changes in tooth count or position that may indicate underlying oral health issues. Such long-term tracking capability could prove invaluable for both subject (i.e. patients) and dental professionals in maintaining optimal oral health.

Proposed embodiment may thus enable objective assessment of oral health care routines by leveraging sensor data from the oral care device, providing valuable insights into the quality and completeness of the routine.

The parameter value may comprise at least one of: a number of teeth, a measure of quality of performance of the oral health care routine, a measure of completion of the oral health care routine, a measure of quality of performance of a subroutine of the oral health care routine, a measure of completion of a subroutine of the oral health care routine, and time duration. By supporting or providing multiple parameter values, such embodiments may allow for a comprehensive evaluation of various aspects of the oral health care routine, enabling targeted improvements and personalized feedback.

Embodiments may further comprise determining a movement speed of the oral health care routine and predicting instances of the dental feature based on the determined movement speed and the routine performance data. This may enhance the accuracy of dental feature detection by accounting for variations in user technique and device movement speed.

Some embodiments may include detecting changes in pressure or motion patterns indicative of transitioning between different dental surfaces. By identifying transitions between dental surfaces, embodiments may more precisely track the progression of the oral health care routine and ensure comprehensive coverage of all areas.

Some embodiments may comprise analysing frequency components of the routine performance data to identify periodic patterns corresponding to individual dental features. This frequency analysis approach provides an alternative means of detecting dental features, potentially improving accuracy in cases where pressure or motion data alone may be insufficient.

The dental feature may comprise an interproximal space between teeth. Focusing on interproximal spaces may allow for accurate tooth counting and ensures attention is given to these critical areas that are often prone to oral health issues.

Embodiments may further involve predicting a number of teeth covered by the oral health care routine based on the identified instances of dental features. This prediction capability may enable assessment of routine completeness and can alert users to potentially missed areas during their oral care routine.

Obtaining routine performance data may comprise sensing, with one or more sensors, at least one of: motion of the oral care device and pressure applied to the oral care device, wherein the one or more sensors comprise at least one of: a pressure sensor, an inertial measurement unit (IMU), or an optical sensor. Utilizing multiple sensor types increases the robustness and accuracy of the data collection process, adapting to various user techniques and environmental conditions.

Embodiments may also include generating a feedback control signal based on the determined parameter value of the oral health care routine. This feedback mechanism may, for example, allow for real-time guidance and post-routine assessment, promoting improved oral care habits over time.

Generating a feedback control signal may comprise comparing the determined parameter value of the oral health care routine against a predetermined threshold value and generating a feedback control signal based on the comparison result. By incorporating predefined thresholds, the method can provide standardized assessments and recommendations, ensuring users meet minimum oral care standards.

According to another aspect of the invention there is provided a computer program product for assessing an oral health care routine performed using an oral care device is provided. The computer program product comprises a computer-readable storage medium having program instructions embodied therewith, the program instructions executable by a processor to cause the processor to perform a method comprising: obtaining routine performance data describing at least one of: motion of the oral care device and pressure applied to the oral care device, during performance of the oral health care routine; analysing the routine performance data to identify one or more instances of a dental feature covered during performance of the oral health care routine; and analysing the identified one or more instances of a dental feature to determine a parameter value of the oral health care routine.

Such a computer program product may enable the implementation of the assessment method in various digital platforms, facilitating widespread adoption and integration with existing oral care technologies.

According to another aspect of the invention there is provided a system for assessing an oral health care routine performed using an oral care device is provided. The system comprises an input interface configured to obtain routine performance data describing at least one of: motion of the oral care device and pressure applied to the oral care device, during performance of the oral health care routine; and a processor arrangement configured to: analyse the routine performance data to identify one or more instances of a dental feature covered during performance of the oral health care routine; and analyse the identified one or more instances of a dental feature to determine a parameter value of the oral health care routine.

Such a system may provide a comprehensive hardware and software solution for implementing the oral health care routine assessment method, enabling seamless integration into various oral care devices and platforms.

Embodiments of the system may further comprise one or more sensors configured to sense at least one of: motion of the oral care device and pressure applied to the oral care device. Incorporating sensors directly into the system ensures consistent and accurate data collection, improving the reliability of the assessment process.

By way of example, the one or more sensors may comprise at least one of: a pressure sensor, an inertial measurement unit (IMU), or an optical sensor. Utilizing multiple sensor types allows the system to capture a diverse range of data points, enhancing its ability to accurately assess various aspects of the oral care routine.

The system may also include a controller configured to generate a feedback control signal based on the determined parameter value of the oral health care routine. This feedback mechanism may enable the system to provide real-time guidance and post-routine assessments, promoting improved oral care habits and user engagement.

Embodiments may be of particular relevance to oral care devices such as Intra-Oral Scanners (IOSs), dental/oral cameras, and electric toothbrushes for example. Thus, according to another aspect of the invention, there may be provided an oral care device comprising a system for according to a proposed embodiment. The oral care device may, for example, comprise an IOS or an electric toothbrush.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a flowchart of a method for assessing an oral health care routine, according to an embodiment of the invention;
Fig. 2 depicts a block diagram of a system for assessing an oral health care routine, in accordance with an embodiment of the invention;
Fig. 3 shows an orthogonal side view of an oral care device incorporating the system of Fig. 2, according to an embodiment; and
Fig. 4 illustrates an example of a computer within which one or more parts of an embodiment may be employed

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to assessing an oral health care routine of a user (such as measuring/imaging procedure, for example). According to proposed concepts, a number of possible solutions may be implemented separately or jointly. That is, although these possible solutions may be described below separately, two or more of these possible solutions may be implemented in one combination or another.

Embodiments of the invention aim to assess the quality and completeness of an oral health care routine performed using an oral care device, such as an intraoral scanner. The system and method leverage sensor data to identify specific dental features, particularly interproximal spaces between teeth, during the performance of the oral health care routine. This data is then analysed to determine a parameter value, such as a quality score, for the oral health care routine. The parameter value may be based on factors such as the number of teeth detected, the consistency of the scanning motion, and the coverage of different areas within the mouth. Further, by comparing the calculated parameter value against predetermined thresholds, the system and method can automatically accept or reject performed routines, ensuring that only high-quality, complete oral health care routines are used for further analysis or diagnosis. This approach aims to enhance the accuracy and efficiency of intraoral scanning or imaging processes for various oral health applications, such as plaque detection, stain assessment, and caries detection.

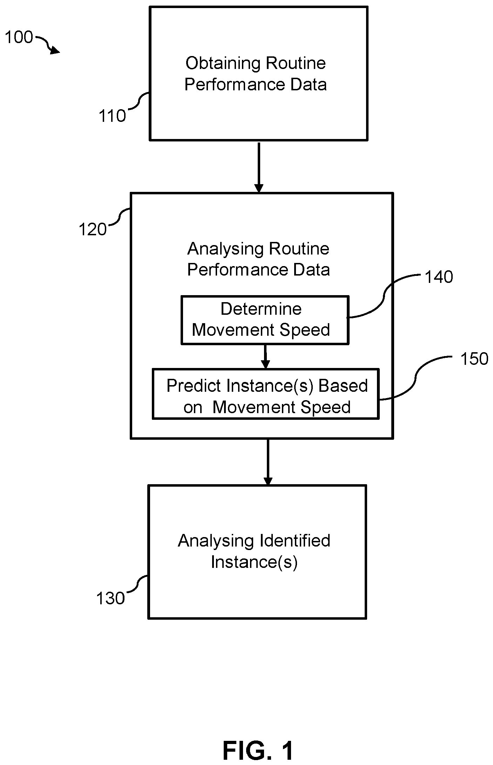

Referring now to Fig. 1, there is depicted a flowchart of a method 100 for assessing an oral health care routine performed using an oral care device according to a proposed embodiment. The flowchart depicts a sequential process where each step leads to the next, showing how the method 100 progresses from data collection to analysis and prediction. The method 100 utilizes the collected performance data to identify dental features and predict their occurrences based on the movement speed of the device.

The method 100 begins with step 110, which involves obtaining routine performance data. This data describes motion of the oral care device or pressure applied to it during the performance of the oral health care routine.

Motion data may be obtained through various sensors that are integrated into the oral care device, such as an inertial measurement unit (IMU). The IMU may be configured to measure the acceleration and angular velocity of the oral care device, providing detailed information about the device's movement patterns during performance of the oral health care routine. Such motion data may be used to identify specific dental features, such as interproximal spaces between teeth.

Similarly, pressure data may be obtained through one or more pressure sensors integrated into the oral care device. The pressure sensors may be configured to measure the force exerted by the user on the oral care device, providing information about the contact between the device and the teeth during performance of the oral health care routine. For example, changes in pressure may indicate transitions between different dental surfaces, such as transitions between teeth surfaces and interproximal spaces (which can be used to count the number of teeth covered (i.e. treated, reached or otherwise exposed to the routine) during performance of the oral health care routine, for example).

Pressure data (i.e. routine performance data) may be used in conjunction with the motion data to enhance the accuracy of the dental feature identification and to provide a more comprehensive assessment of the oral health care routine.

The method 100 then proceeds to step 120, where the routine performance data is analysed. This analysis identifies one or more instances of a dental feature treated during the performance of the oral health care routine.

By way of example, the analysis step 120 in this embodiment involves processing the motion and pressure data obtained from the sensors to detect specific patterns or changes that correspond to the presence of a dental feature. In particular, the analysis detects changes in pressure or motion patterns indicative of transitioning between different dental surfaces, such as transitioning between teeth surfaces and interproximal spaces. These transitions may be used as indicators of the presence of a dental feature, such as an interproximal space between teeth. Interproximal spaces are the spaces between adjacent teeth, and they are a common feature in the dentition.

Utilisation of both pressure and motion data may enhance the accuracy of dental feature identification. By combining the information obtained from the pressure sensors and the IMU, the embodiment may be able to detect subtle changes in pressure and motion patterns that may not be noticeable when using either type of data alone. This combined approach may provide a more comprehensive and accurate assessment, ensuring that all dental features are properly identified (and potentially counted).

The analysis step 120 may utilize machine learning algorithms or other data analysis techniques to identify instances of a dental feature. These algorithms may be trained on large datasets of tooth scans to improve the accuracy of dental feature detection. The algorithms may be configured to recognize specific patterns or changes in the routine performance data that correspond to the presence of a dental feature. For example, the algorithms may recognize a specific pattern of pressure changes or motion changes that indicates the presence of an interproximal space between teeth.

Step 130 involves analysing the identified instance(s) of the dental feature to determine a parameter value of the oral health care routine. This parameter value may be a measure of the quality or completeness of the oral health care routine. For instance, the system may calculate a quality score based on the number of teeth detected, the consistency of the scanning motion, and the coverage of different areas within the mouth. This quality score may serve as a parameter value for assessing the quality and completeness of the oral health care routine.

In particular, by identifying instances of interproximal spaces during the performance of the oral health care routine, the embodiment of Fig. 1 may count the number of teeth treated during the routine. This count may be used as a parameter value for assessing the quality and completeness of the oral health care routine. For example, if the count of teeth is less than the expected number of teeth in a typical dentition, it may be determined that the oral health care routine was not complete and may prompt the user to perform the routine again.

In embodiment of Fig. 1, step 130 includes two sub-steps: step 140 and step 150. In step 140, the movement speed of the oral care device is determined. Following this, step 150 involves predicting instance(s) of the dental feature based on the determined movement speed and the routine performance data.

By way example, step 140 may comprise analysis of the routine performance data to determine the movement speed of the oral care device during the performance of the oral health care routine. This analysis may involve processing the motion data obtained from the sensors to detect specific patterns or changes that correspond to the speed of the device's movement. For instance, the system may analyze the acceleration and angular velocity data obtained from the IMU to calculate the movement speed of the device. This movement speed may provide valuable information about the user's scanning behavior, such as the speed and consistency of the scanning motion.

Step 150 then comprises predicting instances of a dental feature based on the determined movement speed and the routine performance data. This prediction may involve using the movement speed to estimate the timing of the next dental feature, such as the next interproximal space between teeth. For example, if it is determined that the device is moving at a consistent speed, it may be predicted that the next interproximal space will be encountered after a certain period of time based on the typical spacing between teeth. If a change in pressure or motion pattern is detected at the predicted time, it may confirm the presence of an interproximal space and count it as a treated dental feature.

Some implementations of such an embodiment may adjust predictions based on variations in the movement speed of the oral care device. For instance, if it is detected that the device is moving faster or slower than expected, the predicted timing of the next dental feature may be adjusted accordingly. This flexibility may allow accurate teeth counting and quality assessment of the oral health care routine, even when the user's behaviour varies.

Furthermore, the determined movement speed and the routine performance data may be used to predict the size of the teeth being scanned. As teeth have a typical size, knowing the speed can predict the expected timing of the next interproximal space signal. If the signal comes at a very different time point, it may be neglected as a count. Knowing the scan speed and the time between the interproximal space signals can also add another benefit of this invention which is to measure the tooth size. This in turn could indicate in which part the teeth are counted: e.g. when large teeth are detected the scanner may be on molars while small teeth would indicate the lower jaw incisors. This may further enhance the accuracy of the tooth counter and the overall quality assessment of the oral health care routine.

Variations to the analysis steps detailed above for the embodiment of Fig. 1 may be employed.

For example, some embodiments may analyse the routine performance data by detecting changes in motion patterns indicative of transitioning between different dental surfaces. For instance, embodiments may monitor motion data obtained from an inertial measurement unit (IMU) integrated into the oral care device. As the device moves across the teeth and into the interproximal spaces, there may be noticeable changes in the device's acceleration and angular velocity. These changes in motion patterns may be used as indicators of the presence of a dental feature, such as an interproximal space between teeth. By detecting these changes in motion patterns, the system may be able to identify instances of a dental feature and count the number of teeth treated during the oral health care routine.

Other embodiments may analyse the routine performance data by analysing frequency components to identify periodic patterns corresponding to individual dental features. This analysis may involve processing the motion and pressure data obtained from sensors to detect specific patterns or changes that correspond to the presence of a dental feature. For instance, frequency analysis on the motion data may be performed to identify periodic patterns that correspond to the repetitive motion of the device moving across the teeth and into the interproximal spaces. These periodic patterns may be used as indicators of the presence of a dental feature, such as an interproximal space between teeth. By detecting these periodic patterns, embodiments may be able to identify instances of a dental feature and count the number of teeth treated during the oral health care routine.

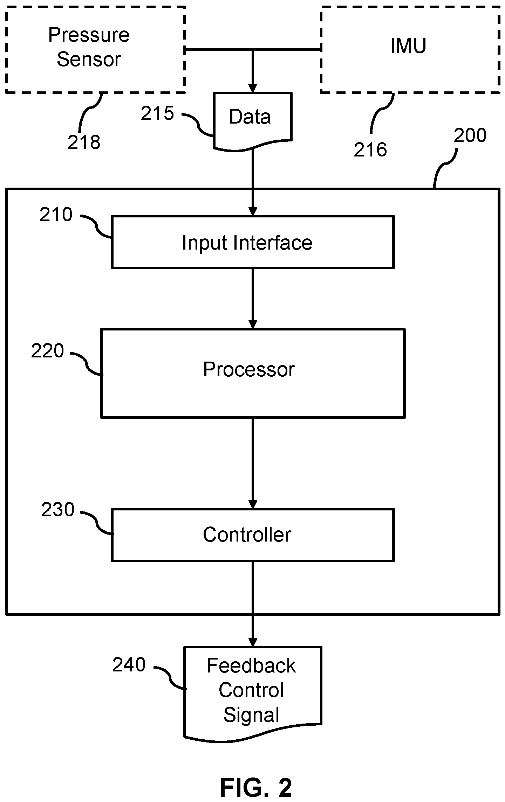

Referring now to Fig. 2, there is depicted a block diagram of a system 200 for assessing an oral health care routine performed by user (using an oral care device). The system 200 is designed to process sensor data from the oral care device, analyse the performance of the oral health care routine, and provide feedback to improve the user's oral care technique.

The system 200 has an input interface 210 in communication with a pressure sensor 218 and an Inertial Measurement Unit (IMU) 216 so as to receive routine performance data 215. The pressure sensor 218 is configured to measure the force exerted by the user on the oral care device, providing information about the contact between the device and the teeth. The IMU 216 is configured to measure the acceleration and angular velocity of the oral care device, providing detailed information about the device's movement patterns. The input interface 210 is thus configured to receive data describing the motion of the oral care device and the pressure applied to the device during the performance of the oral health care routine. The interface may also be configured to receive data from an optical sensor, for example.

The input interface 210 forwards the received data 215 from the sensors 216, 218 to a processor 220 of the system 200. The processor 220 analyses the received data to identify instances of dental features treated during the performance of the oral health care routine and determines one or more parameter values of the oral health care routine.

In particular, the processor 220 is configured to analyse the receive routine performance data 215 to identify specific dental features, such as interproximal spaces between teeth. The processor 220 is also configured to analyse the identified one or more instances of a dental feature to determine a parameter value of the oral health care routine. Here, the processor 220 analyses the identified instances of a dental feature to predict a number of teeth treated by the oral health care routine. This prediction involves counting the number of detected dental features, such as interproximal spaces, and using this count to estimate the number of teeth treated during the routine. For example, if the system detects a certain number of interproximal spaces, it may predict that a corresponding number of teeth have been treated during the routine. This predicted number of teeth may serve as a parameter value for assessing the completeness of the oral health care routine.

By way of example, the processor 220 may utilize machine learning algorithms or other data analysis techniques to analyse the identified instances of a dental feature and to determine parameter values of the oral health care routine. These algorithms may be trained on large datasets of tooth scans to improve the accuracy of dental feature detection and parameter value determination. For instance, the algorithms may be configured to recognize specific patterns or changes in the routine performance data that correspond to the presence of a dental feature, and to calculate parameter values based on these recognized patterns or changes.

The parameter value may be based on factors such as the number of teeth detected, the consistency of the scanning motion, and the coverage of different areas within the mouth.

A parameter value that represents the measure of quality of performance of the oral health care routine may be determined based on various factors such as the consistency of the scanning motion, the coverage of different areas within the mouth, and the accuracy of the dental feature detection. For example, if the processor 220 detects a consistent scanning motion and a comprehensive coverage of all areas within the mouth, it may assign a high-quality score to the oral health care routine.

A parameter value that represents the measure of completion of the oral health care routine may be determined based on the number of teeth detected and the coverage of different areas within the mouth. For instance, if the processor 220 detects that all teeth have been scanned and all areas within the mouth have been covered, it may assign a high completion score to the oral health care routine.

A parameter value that represents the time duration may be determined based on the total time taken to perform the oral health care routine or a subroutine thereof. For instance, if the processor 220 detects that the oral health care routine or the subroutine has been performed within a predetermined time limit, it may assign a high time efficiency score to the routine or the subroutine.

Information describing the determined parameter value(s) is then passed to a controller 230, which generates a feedback control signal 240 based on the determined parameter value(s). This feedback control signal 240 can be used to provide guidance or instructions to the user of the oral care device. For example, the feedback control signal may be used to guide the user during the performance of the oral health care routine or to provide feedback to the user after the completion of the routine. For instance, if it is determined that the oral health care routine was not complete, or that the quality of the routine was below a certain threshold, the controller 230 may generate a feedback control signal instructing the user to perform the routine again or to focus on certain areas within the mouth. Such post-routine feedback may help the user to understand the effectiveness of their oral health care routine and to make necessary adjustments for future routines.

In this embodiment, the controller 230 is configured to compare the determined parameter value of the oral health care routine against a predetermined threshold value. If the determined parameter value is below the threshold value, the controller generates a feedback control signal indicating that the oral health care routine was not complete or that the quality of the routine was below the desired level. By comparing the calculated parameter value against predetermined thresholds, the processor arrangement may be configured to automatically accept or reject performed routines, ensuring that only high-quality, complete oral health care routines are used for further analysis or diagnosis.

The feedback control signal may be communicated to the user through various means, such as visual indicators on the oral care device, auditory signals, or haptic feedback. For instance, LED indicators on the oral care device visually communicate the feedback control signal to the user. Embodiments may also use auditory signals, such as beeps or voice prompts, to audibly communicate the feedback control signal to the user. In other examples, haptic feedback may be employed, such as vibrations, to physically communicate the feedback control signal to the user. Such multi-modal feedback approach may enhance the user's understanding of the feedback control signal and may help the user to improve the quality and completeness of the oral health care routine.

In another example, the controller 230 may generate a feedback control signal instructing the user to slow down the scanning motion, to apply more pressure to the oral care device, or to focus on certain areas within the mouth. This real-time feedback may help the user to improve the quality and completeness of the oral health care routine, ensuring that all dental features are properly scanned and treated.

The embodiment of Fig. 2 does not include the sensors. That is, the system of Fig. 2 is configured to leverage data from pre-existing sensors provided in/with the oral care device.

In alternative embodiments, the system may include one or more sensors configured to generate routine performance data. These sensors may be integrated into the oral care device and may include, for example, a pressure sensor, an inertial measurement unit (IMU), and/or an optical sensor.

The optical sensor may be configured to detect light reflected from the teeth, providing information about the shape and spacing of the teeth. This optical data may provide information about the shape and spacing of the teeth. For instance, changes in light reflection may indicate transitions between different dental surfaces, such as transitions between teeth surfaces and interproximal spaces. These transitions may be used as indicators of the presence of a dental feature, such as an interproximal space between teeth.

In some cases, the system may utilize capacitive sensors to detect changes as the oral care device moves across teeth and into interproximal spaces. These capacitive sensors may be integrated into the oral care device and may be configured to detect slight changes in capacitance as the device moves across the teeth and into the interproximal spaces. These changes in capacitance may be used as indicators of the presence of a dental feature, such as an interproximal space between teeth. By detecting these changes in capacitance, the system may be able to identify instances of a dental feature and count the number of teeth treated during the oral health care routine. This approach may provide a non-contact method for dental feature identification, enhancing the user's comfort during the oral health care routine and potentially improving the accuracy of the tooth count.

Further, in some embodiments, the sensors may be integrated into different parts of the oral care device. For example, a pressure sensor may be integrated into a handle of the device, while an IMU and/or an optical sensor may be integrated into a brush head or scanning module of the device. Such a configuration may allow for more accurate and comprehensive sensing of the oral health care routine.

In some aspects, the system may determine various parameter values based on the analysis of the identified instances of a dental feature. These parameter values may include a number of teeth, a measure of quality of performance of the oral health care routine, a measure of completion of the oral health care routine, a measure of quality of performance of a subroutine of the oral health care routine, a measure of completion of a subroutine of the oral health care routine, and time duration.

Further, in some cases, the system may adjust its parameter value determination based on variations in the routine performance data. For instance, if the system detects that the device is moving faster or slower than expected, or if the pressure applied to the device varies significantly, it may adjust the calculated parameter values accordingly. This flexibility may allow the system to accurately assess the quality and completeness of the oral health care routine even when the user's scanning behaviour varies.

In some embodiments, the system may predict the number of teeth treated based on the identified interproximal spaces. This prediction may involve measuring the time between the signals corresponding to the detected interproximal spaces and the scan speed of the oral care device. For instance, the system may calculate the scan speed based on the motion data obtained from the sensors, such as the IMU. By knowing the scan speed and the time between the interproximal space signals, the system may predict the size of the teeth being scanned. As teeth have a typical size, this prediction may provide an estimate of the number of teeth treated during the oral health care routine. For example, if large teeth are detected, the system may predict that the scanner is on molars, while small teeth would indicate the lower jaw incisors. This feature may further enhance the accuracy of the tooth counter and the overall quality assessment of the oral health care routine.

In some embodiments, the system may integrate the tooth counting system with existing dental records for more accurate quality assessments. For instance, the system may compare the counted teeth to known information about the patient's dentition, such as the number of teeth, presence of implants or bridges, and other dental features. By comparing the counted teeth with the existing dental records, the system may provide more accurate quality assessments and potentially identify changes in oral health over time. For example, if the system detects a discrepancy between the counted teeth and the existing dental records, it may indicate that a tooth has been lost or a new dental implant has been added. This feature may provide valuable insights into the patient's oral health status and may help in early detection of oral health issues.

By way of further example of other potential modifications, an embodiment may implement adaptive power management techniques by dynamically adjusting the sensor sampling rate based on the detected scanning speed. This approach may involve monitoring the motion data obtained from the sensors, such as the IMU, to determine the scanning speed of the oral care device. The sensor sampling rate may then be adjusted based on the determined scanning speed, increasing the sampling rate when rapid movements are detected and decreasing the sampling rate during slower, more deliberate scanning. This adaptive power management approach may help to balance power consumption with the need for accurate tooth counting and quality assessment.

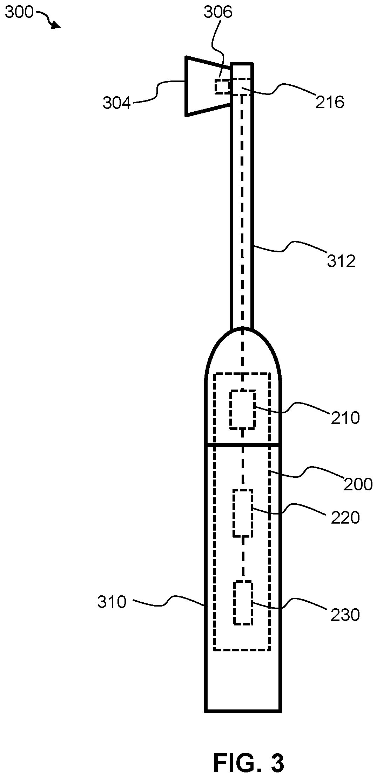

Fig. 3 illustrates a side view of an oral care device 300 incorporating the system 200 of Fig. 2. The oral care device 300 is an intraoral scanner which comprises a handle section 310 atop which is an elongated arm 312.

At the distal end of the arm is a conical section 304 which houses an optical sensor 306 for performing intraoral scanning functions. Also provided at the distal end of the arm 312, adjacent the optical sensor 306, is an IMU 216. The IMU 216 is configured to measure the acceleration and angular velocity of the oral care device, providing detailed information about the device's movement patterns.

The system 200 is integrated within the handle section 310. The input interface 210 of the system is positioned near the top of the handle section 310, to receive routine performance data from the sensors 216, 306. Below the input interface 210 is the processor 220, which is responsible for analysing the routine performance data. The controller 230 of the system is situated at the bottom of the handle 300.

Connections link the sensors 216, 306 to the input interface 210, the input interface 210 to the processor 220, and the processor 220 to the controller 230. The connections thus facilitate the flow of data and signals within the system 200.

The arrangement of components within the oral care device 300 allows for efficient data processing and control of the oral care device according to the proposed concept(s) for assessing an oral health care routine performed using the oral care device. The input interface 210 receives routine performance data from the sensors 306 and 216, the processor 220 analyses this data, and the controller 230 generate appropriate feedback or control signals based on the result(s) of the analysis.

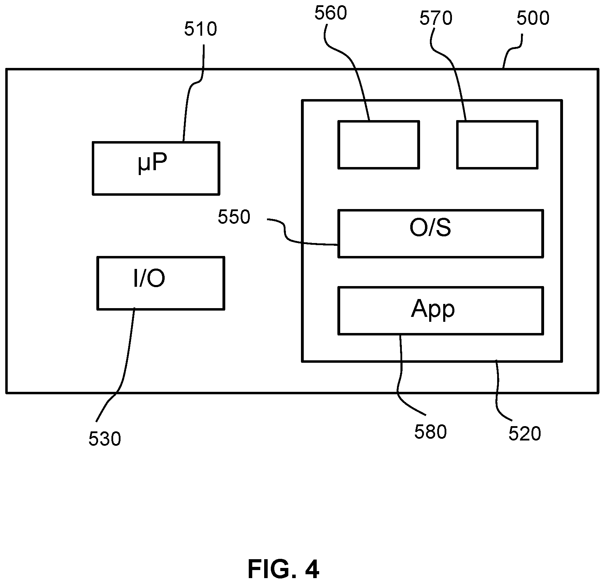

Fig. 4 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The embodiments of the Figures may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for assessing an oral health care routine performed using an oral care device, the method comprising:
obtaining (110) routine performance data (215) describing at least one of: motion of the oral care device; and pressure applied to the oral care device, during performance of the oral health care routine;
analysing (120) the routine performance data to identify one or more instances of a dental feature treated during performance of the oral health care routine; and
analysing (130) the identified one or more instances of a dental feature to determine a parameter value of the oral health care routine.

2. The method of claim 1, wherein the at least one parameter value comprises at least one of: a number of teeth; a measure of quality of performance of the oral health care routine; a measure of completion of the oral health care routine; a measure of quality of performance of a subroutine of the oral health care routine; a measure of completion of a subroutine of the oral health care routine; and time duration.

3. The method of claim 1 or 2, wherein analysing (120) the routine performance data comprises:
determining (140) a movement speed of the oral health care routine; and
predicting (150) instances of the dental feature based on the determined movement speed and the routine performance data.

4. The method of claim 1 or 2, wherein analysing (120) the routine performance data comprises:
detecting changes in pressure or motion patterns indicative of transitioning between different dental surfaces.

5. The method of claim 1 or 2, wherein analysing (120) the routine performance data comprises:
analysing frequency components of the routine performance data to identify periodic patterns corresponding to individual dental features.

6. The method of any of claims 1 to 5, wherein the dental feature comprises an interproximal space between teeth.

7. The method of claim 6, wherein analysing (130) the identified one or more instances of a dental feature to determine a parameter value of the oral health care routine comprises predicting a number of teeth covered by the oral health care routine.

8. The method of any of claims 1 to 7, wherein obtaining (110) routine performance data comprises sensing, with one or more sensors, at least one of: motion of the oral care device; and pressure applied to the oral care device, and wherein the one or more sensors comprise at least one of: a pressure sensor (218), an inertial measurement unit, IMU 216, or an optical sensor (306).

9. The method of any of claims 1 to 8, further comprising:
generating a feedback control signal (240) based on the determined parameter value of the oral health care routine.

10. The method of claim 9, wherein generating a feedback control signal (240) comprise:
comparing the determined parameter value of the oral health care routine against a predetermined threshold value; and
generating a feedback control signal (240) based on the comparison result.

11. A computer program product for assessing an oral health care routine performed using an oral care device (300), the computer program product comprising a computer-readable storage medium having program instructions embodied therewith, the program instructions executable by a processor to cause the processor to perform a method comprising:
obtaining (110) routine performance data describing at least one of: motion of the oral care device; and pressure applied to the oral care device, during performance of the oral health care routine;
analysing (120) the routine performance data to identify one or more instances of a dental feature covered during performance of the oral health care routine; and
analysing (130) the identified one or more instances of a dental feature to determine a parameter value of the oral health care routine.

12. A system (200) for assessing an oral health care routine performed using an oral care device (300), the system comprising:
an input interface (210) configured to obtain routine performance data describing at least one of: motion of the oral care device; and pressure applied to the oral care device, during performance of the oral health care routine; and
a processor (220) arrangement configured to:
analyse the routine performance data to identify one or more instances of a dental feature treated during performance of the oral health care routine; and
analyse the identified one or more instances of a dental feature to determine a parameter value of the oral health care routine.

13. The system of claim 12 further comprising one or more sensors (216,218) configured to sense at least one of: motion of the oral care device (300); and pressure applied to the oral care device and optionally, wherein the one or more sensors (216, 218) comprise at least one of: a pressure sensor (218), an inertial measurement unit, IMU (216), or an optical sensor (306).

14. The system of any of claims 12 to 13, further comprising:
a controller (230) configured to generate a feedback control signal (240) based on the determined parameter value of the oral health care routine.

15. An oral care device comprising a system according to any of claims 12-14 for assessing an oral health care routine performed using the oral care device, and optionally wherein the oral care device comprises an Intra-Oral Scanner (IOS).
